Europäisches Patentamt

European Patent Office

Office européen des brevets

(11) Publication number: **0 305 279 B1**

(12) **EUROPEAN PATENT SPECIFICATION**

(45) Date of publication of patent specification: 29.03.95

(51) Int. Cl.⁶: **C12N 15/03**, C12N 15/31, C12N 1/20, A61K 39/09, //(C12N1/20,C12R1:46), (C12N1/20,C12R1:42)

(21) Application number: 88402132.0

(22) Date of filing: 22.08.88

(54) **Therapeutic compositions against streptococcal infections, transformed hosts, methods of immunization and genetically engineered products.**

(30) Priority: 24.08.87 US 88626

(43) Date of publication of application:
01.03.89 Bulletin 89/09

(45) Publication of the grant of the patent:
29.03.95 Bulletin 95/13

(84) Designated Contracting States:
**AT BE CH DE ES FR GB IT LI LU NL SE**

(56) References cited:
**WO-A-85/00832**
**US-A- 4 597 967**

**Nature, vol. 292, no. 5822, July 30, 1981 (NEW YORK, London) E.H. BEACHEY et al.: pages 457-459**

(73) Proprietor: **Beachey, Edwin H.**
**2909 Central Avenue**
**Memphis, TN 38111 (US)**

Proprietor: **Poirier, Thomas P.**

1542 Tutwiler Avenue
Memphis, TN 38107 (US)

Proprietor: **Kehoe, Michael A.**
**14 Springhouse Lane**
**Ebchester**
**Co. Durham DH8 COF (GB)**

(72) Inventor: **Beachey, Edwin H.**
**2909 Central Avenue**
**Memphis, TN 38111 (US)**
Inventor: **Poirier, Thomas P.**
**1542 Tutwiler Avenue**
**Memphis, TN 38107 (US)**
Inventor: **Kehoe, Michael A.**
**14 Springhouse Lane**
**Ebchester**
**Co. Durham DH8 COF (GB)**

(74) Representative: **Orès, Bernard et al**
**Cabinet ORES**
**6, Avenue de Messine**
**F-75008 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

This invention relates to immunization against Streptococcal infections, more particularly against group A streptococcal infections.

The invention has broad and important implications. As far as is known by the inventor, there is provided for the first time a biological living vehicle which carries a protective antigen of a virulent bacteria which antigen is effective to immunize against infections caused by the virulent bacteria. It is contemplated that the embodiments here disclosed are applicable to broader and more far reaching applications, methods and uses.

The search for a safe and effective vaccine against those strains of group A streptococci that trigger rheumatic fever and rheumatic heart disease has been ongoing for more than sixty hears. Lancefield, "Current Knowledge of Type-Specific M Protein Antigens to Group A Streptococci, " J. Immunol, Vol. 89, pp. 307 - 313 (1962). It has been reported that few bacterial species have been subjected to more intensive investigation during the century than Streptococcus pyogenes or the group A streptococcus. As conviction grew that this organism was first the principal, and then the exclusive agent for acute rheumatic fever, researchers sought with much determination and effort to dissect out the product of the bacterium whose toxic or antigenic components might touch off the rheumatic process. For a thorough study of rheumatic fever and streptococcal infection See Stollerman, Rheumatic Fever and Streptococcal Infection (New York Clinical Cardiological Monographs, Grune and Stratton, 1975). The central role of M protein in immunity against group A streptococci has been reviewed by Stollerman. Reference is also made to Beachey and Seyer, "Primary Structure and Immunochemistry of Group A Streptococcal M Proteins," Seminars in Infectious Disease, Vol. 4, pp. 401 - 410 (J.B. Robbins, J.C.Hill and J.C. Sadoff, eds., Georg Thiemeverlag, pub., New York and Stuttgart, 1982).

Most efforts to develop a vaccine were frustrated by severe toxic reactions to almost any Streptococcal product introduced into the human host. Some of these products have been shown to give rise to antibodies that cross react with host tissues, especially the heart. Kaplan and Meyerserian, "An Immunological Cross-Reaction Between Group A Streptococcal Cells and Human Heart Tissue, "Lancet, Vol. i, pp 706 - 710 (1962) ; Zabriskie and Freimer, "An Immunological Relationship Between the Group A Streptococcus and Mammalian Muscle," J. Exp. Med, Vol. 124, pp. 661 - 678 (1966). Although it has been long established that the M Protein on the surface of group A Streptococcus contains the protective antigens of these organisms, the fear has been that the isolated M protein may be associated with potentially harmful tissue cross-reactive antigens that give rise to rather than prevent, rheumatic fever. This fear has been perpetuated by the finding that certain rheumatogenic Streptococci produce M proteins that are closely associated with a heart cross-reactive antigen. Kaplan, "Immunologic Relation of Streptococcal and Tissue Antigens. I. Properties of an Antigen in Certain Strains of Group A Streptococci Exhibiting an Immunologio Cross-Reaction with Human Heart Tissue," J. Immunol. Vol. 90, p. 595 (1963). Indeed, recently it has been established that one of the M protein molecules contains, within its covalent structure, an epitope that elicits a protective anti-Streptococcal antibody that also cross-reacts with a sarcolemmal protein of human heart tissue . Dale and Beachey, "Protective Antigenic Determinant of Streptococcal M Protein Shared with Sarcolemmal Membrane Protein of Human Heart," J. Exp. Med., Vol. 156, pp. 1165 - 1176 (1982).

United States Patent No. 4,284,537, to E. Beachey, issued August 18, 1981, disclosed the amino acid sequence of two peptide fragments derived from type 24 M protein. It also disclosed that each of these natural fragments, when covalently linked to a carrier such as a polylysine, was able to elicit type-specific opsonic antibodies effective against Streptococcus pyogenes. Each of these fragments was a natural extract, and each contained 35 amino acids.

United States Patent No. 4,454,121, to E. Beachey, issued June 12, 1984, disclosed a synthetic peptide (S-CB7) and that one of the protective determinants is located in a specific fragment of S-CB7 of type 24 M protein which contains only twelve amino acid residues (S-CB7(18-29)). S-CB7, as described, differs from the native CB-7 fragment in that the COOH-terminal residue of S-CB7 is methionine, in contrast to homoserine. The specification also teaches and described covalently linked conjugates of S-CB7 and appropriate hapten carriers, natural, like BSA or OVA or synthetic, like polylysine. Further details about this work have been published in Nature on July 30, 1981, by Beachey et al, 292, pages 457 - 459.

United States Patent No. 4,521,334, entitled "Synthetic Polypeptide Fragments," to Edwin H. Beachey issued June 4, 1985, discloses the amino acid sequence of three peptide fragments CB3, CB4 and CB5, and 35 and 37 amino acid sequences of type 24 M which contain antigenic determinants corresponding to the antigenic determinants contained in CB3 - CB7. United States Patent No. 4,597,967 entitled "Synthetio Polypeptide Fragments," to Edwin H. Beachey, issued July 1, 1986, disclosed that these fragments, when covalently linked to a carrier such as polylysine, are able to elicit type-specific opsonic antibodies effective

2

against Streptococcus pyogenes.

United States Application Serial No. 739,963 entitled "Biologically Active Hybrid Peptides of Streptococcal M Protein and Compositions and use" to Edwin H. Beachey et al filed May 31, 1985 disclosed peptide sequences containing fragments of M5, M6, and M24 proteins which are able to elicit opsonic and bactericidal antibodies to Streptococcus pyogenes which are not serologically cross-reactive with tissue antigens of the human or host heart. United States Application Serial No. 839,750 entitled "Synthetic M Proteins-Streptococci Type 6" to Edwin H. Beachey, et al filed March 14, 1986 disclosed the synthesis of type M6 protein antigen conjugates. United States Application Serial No. 858,436 entitled "Localization of Protective Epitopes of the Amino Terminus of Type S Streptococcal M Protein," to Edwin H. Beachey, et al filed May 1, 1986 disclosed the synthesis of Type M5 protein antigen conjugates.

The above patents disclose small peptide fragments which are immunogenic and contribute to the development of a safe and effective vaccine against those streptococcal infections that initiate fevers and rheumatic heart disease. The approach was that very small peptides would permit disposal of a large portion of the M protein molecule and therefore, should reduce the chances of eliciting immunological cross-reactions against host tissues. See for instance the above-referred to United States Patent 4,454,121 Columns 1 and 2.

For additional information regarding type specific protective immunity evoked by synthetic peptides of Streptococcus pyogenes M protein, See, Beachey, et al., "Type Specific Protective Immunity Evoked by Synthetic Peptide of Streptococcus pyogenes M Protein," Nature, Vol. 292, No. 5822, pp 457 - 459 (July 30, 1981).

For additional literature in this field See Hasty, et als, "Hybridomas Antibodies Against Protective and Non-Protective Antigenic Determinants of a Structurally Defined Polypeptide Fragment of Streptococcal M Protein, "J. Exp. Med. Vol. 155, p 1010 (April 1982) ; and Hopp and Woods, "Prediction of Protein Antigenic Determinants from Amino Acid Sequences, "Proc. Natl. Acad. Sci. USA, Vol. 78, N°. 6, pp. 3824 - 28 (June 1981).

Not withstanding these advances, there remains a serious need, as yet unfilled for an orally administrable vaccine incorporating these non-cross-reactive immunogenic polypeptides. By administering these peptides in the form of an attenuated non virulent recombinant bacterium capable of their synthesis, the present invention marks another forward step and provides another advance in the medical sciences, particularly in the control of Streptococcal infections.

Numerous serotypes of M proteins are known, coded on genes that are alleles of each other. Each serotype corresponds to a different strain of S. pyogenes and these serotypes differ only by their amino terminal sequences.

An object of this invention is a non-virulent live bacterium transformed by an heterologous nucleotide sequence coding for and expressing at least a serotype-specific streptococcal M protein immunizing antigen, which is effective to elicit opsonic antibodies against streptococcal infections without eliciting antibodies which are cross reactive with human heart tissue antigens, for use as a vaccine.

A transformed bacterium which is suitable for obtention of the vaccine of the invention results from the transformation of a non-virulent host bacterium, particularly a non-virulent enteric bacterium, of a strain capable of invasion, but which is a mutant strain incapable of causing disease. This non-virulent bacterium will not colonize in the subject to be immunized, but will multiply to the limited extent necessary to elicit the antibodies.

The invention also encompasses a non-virulent bacterium transformed by an heterologous nucleotide sequence as defined above and which :

- is selected from the group consisting of Salmonella and Streptococcus ; and/or
- has a mutational variation causing a nutritional deficiency, i.e exhibits a nutritional marker for metabolites not found in the tissues of the subject to be immunized.

Said transformed non-virulent live bacterium can advantageously be used as a vaccine, according to the invention.

According to an embodiment of the invention, the non-virulent bacterium transformed is of the genus Salmonella, specifically the typhimurium species, which carries a plasmid which encodes an M protein gene, more specifically the type 5 M protein gene.

Preferably, said Salmonella typhimurium strain is Aro⁻Sal. typhimurium. SL3261.

According to yet another embodiment, the non-virulent bacterium transformed is of the non-group A species of the genus Streptococcus, preferably Streptococcus sanguis, or Streptococcus mutans.

According to an embodiment of the invention, the heterologous nucleotide sequence expressing at least a streptococcal M protein immunizing antigen is contained in a plasmid.

According to another embodiment, the nucleotide sequence expresses a streptococcal M protein immunizing antigen effective to elicit opsonic antibodies against Streptococcal infections for Streptococcal serotypes 5, 6 and 24. It can also express a multivalent Streptococcal M protein antigen which is effective to elicit opsonic antibodies against more than one serotype M Streptococcal strain.

Preferably, said Streptococcal M protein antigen is effective to elicit opsonic antibodies and confer immunity against the type 5 Streptococci.

It is a noteworthy aspect of the invention that the immunity conferred to the patient is systemic and is conferred comparatively rapidly and completely even when the transformed live bacterium is administered orally. However, local immunity is also provided.

An important aspect of the invention is to provide immunity against oral, and also against parenteral (such as intranasal and intraperitoneal) infections of the streptococcal type.

Another object of the invention is a therapeutic composition which comprises in an amount effective to elicit opsonic antibodies and to confer immunity against streptococcal infections, the transformed non-virulent bacterium containing the heterologous nucleotide sequence described above.

Advantageously, said compositions are for oral administration, although they can also be provided for other administrations, including the parenteral route.

The compositions of the invention are non-virulent and very well tolerated by the subject to which they are administered.

Other objects of the invention will become apparent from the description which follows. Other features and advantages of the invention will appear from the examples which follow and by referring to the appended drawing in which :

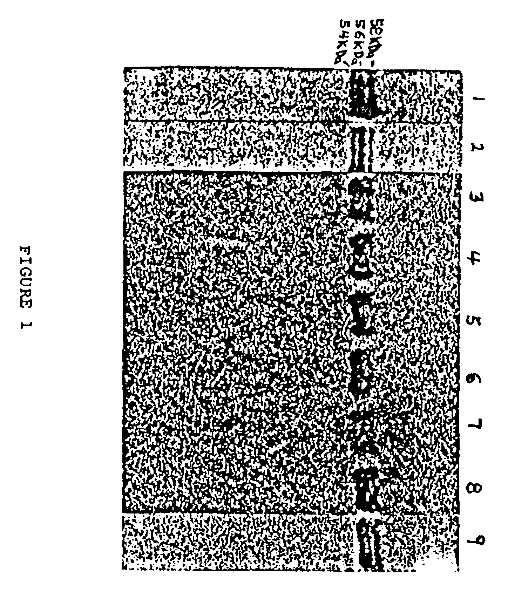

Fig. 1 shows Immunoblot analysis of type 5M protein expressed by pMK 207-transformed Salmonella typhimurium LB5000 (Lane 1) and SL3261 (Lane 2-9).

In addition to the patents and other publications mentioned above, other prior art which has been taken into consideration in the description of this invention includes :

Beachey, et als., "Repeating Covalent Structure and Protective Immunogenicity of Native and Synthetic Polypeptide Fragments of Type 24 Streptococcal M Protein," J. Biol. Chem, Vol. 258, No. 21 pp 13,250 - 13,257 (1983).

van de Rijn, et als., "Group A Streptococcal Antigens Cross-Reactive with Myocardium," J. Exp. Med., Vol. 146, pp. 579 - 599 (1977).

van de Rijn, et als., "Immunochemical Analysis of Intact M Protein Secretd From Cell Wall-Less Streptococci," Infect. Immun., Vol. 32, pp. 86 - 91 (1981).

Edman and Begg, "A Protein Sequenator," European J. Biochem. Vol.1, pp. 80 - 91 (1967).

Phillips, et als., "Streptococcal M Protein ; Helical Coiled - Coil Structure and Arrangement on the Cell Surface," Proc. Natl. Acad. Sci. USA, Vol. 78, No. 8, pp. 4689 - 4693 (August 1981).

Laver, et als, "Antigenic Drift in Type A Influenza virus : Peptide Mapping and Antigenic Analysis of A/PR/8/34(HONI) Variants Selected With Monoclonal Antibodies," Proc. Natl. Acad. Sci. USA, Vol. 76, No. 3, pp. 1425 - 1429 (March 1979).

Atassi, "Antigenic Structure of Myoglobin : The Complete Immunochemical Anatomy of a Protein and Conclusions Relating to Antigenic Structures of proteins," Immunochemistry, Vol. 12, pp. 423 - 438 (1975).

Kabat, Structural Concepts in Immunology and Immunochemistry, pp. 89 - 100 (Holt, Rhinehart & Winston, New York, (1968).

Nisonoff, Methods in Immunology and Immunochemistry, Vol. 1, pp. 120 - 187 (1977).

Munoz, Methods in Immunology and Immunochemistry, Vol. 3, pp. 146 - 160 (1970).

Manjula and Fischetti, "Tropomyosin-like Seven Residue Periodicity in Three Immunologically Distinct Streptococcal M Proteins and its Implications for the Antiphagocytic Property of the Molecule," J. Exp. Med., Vol. 155, pp. 695 - 708 (1980).

Beachey and Stollerman, "Toxic Effects of Streptococcal M Protein on Platelets and Polymorphonuclear Leukocytes in Human Blood," J. Exp. Med. Vol. 134, pp. 351 - 365 (1971).

Dale et als., "Heterogenecity of Type-Specific and Cross-Reactive Antigenic Determinants Within a Single M Protein of Group A Streptococci," J. Exp. Med. Vol.155 pp. 1026 - 1038 (1980).

Beachey, et als. "Purification and Properties of M Protein Extracted from Group A Streptococci with Pepsin : Covalent Structure of the Amino Terminal Region of Type 24 M Antigen," J. Exp. Med. Vol. 145, pp. 1469 - 1483 (1977).

Beachey, et als., "Primary Structure of Protective Antigens of Type 24 Streptococcal M Protein," J. Biol. Chem., Vol. 255, pp. 6284 - 6289 (1980).

Beachey, et als., "Repeating Covalent Structure of Streptococcal M Protein," Proc. Natl. Acad. Sci. USA Vol. 75, pp. 3163 - 3167 (1978).

Beachey, et als., "Human Immune Response to Immunization with a Structurally Defined Polypeptide Fragment of Streptococcal M Protein" J. Exp. Med., Vol. 150, pp. 862 (1979).

Brown, et al., "An Attenuated aroA Salmonella typhimurium Vaccine Elicits Humoral and Cellular Immunity to Cloned -Galactosidase in Mice," The Journal of Infectious Diseases, Vol. 155, No. 1 (January 1987). This publication discusses salmonella typhimurium strain SL3261 and an attenuated aroA vaccine strain, which was used as a carrier for the plasmid pXY411.

Another publication of interest is Hoiseth and Stocker, "Aromatic-dependent Salmonella Typhimurium are Non-Virulent and Effective as Live Vaccines," Nature, Vol. 291 (May 21, 1981) ; Smith, et al., "Aromatic-Dependent Salmonella Typhimurium are Non-Virulent and Effective as Live Vaccines," Am. J. Vet. Res., Vol. 45, No. 1 (January 1984) ; Smith et al., "Vaccination of Calves Against Salmonella Dublin with Aromatic-Dependent Salmonella Typhimurium," Am. J. Vet. Res., Vol. 15, No. 11 (November 1984) ; Maskell, et al., "Attenuated Salmonella Typhimurium as Live Oral Vaccines and Carriers for Delivering Antigens to the Secretory Immune System," Vaccines 86 (Cold Spring Habor Laboratory 1986).

Patents in the genetic engineering field which are of general interst include :

United States Patent No. 4,428,941 entitled "Nucleotide Sequence Coding the Surface of the Hepatitis B Virus, Vector Containing Said Nucleotide Sequence, Process Allowing the Obtention Thereof and Antigen Obtained Thereby," issued to Francis Galibert, et als., on January 31, 1984; United States Patent No. 4,518,584 entitled "Human Recombinant Interleukin-2 Muteins," issued to Mark, et als., on May 21, 1985 ; United States Patent No. 4,588,585 entitled "Human Recombinant Cysteine Depleted Interferon-B Muteins," issued to Mark, et als., on May 13, 1986 ; and United States Patent No. 4,625,252 entitled "Recombinant Bacterial Plasmids Containing the Coding Sequences of Insulin Genes," issued to Rutter, et als., on March 24, 1987.

United States Patent No. 4,666,846 entitled "Novel Cloning Vectors Containing Selectable Genetic Markers for Use in Streptomyces and Related Organisms," issued to Fayerman, et als. on May 19, 1987 ; and United States Patent No. 4,666,847 entitled "Recombinant DNA Means and Methods," issued to Alford, et als., on May 19, 1987.

It is preferable that the plasmid which encodes the M protein gene be cloned first and expressed in Escherichia coli. Any other enteric bacilli of the coliform group such as Klebsiella or Enterobacter can be used, but normally E. coli is preferred. Thereafter the plasmid carrying the M gene is isolated and purified and then a construct is built to transform the desired non-virulent bacteria, such as the aroA-S. typhimurium (SL3261). It is to be noted that this mutant strain exhibits a nutritional marker both for PABA and 2,3-DHB. See Brown et al., cited above. It is to be noted that another desired specie of S. typhimurium is recA⁻S. typhimurium, particularly strain Ty21a. See Clements, et al., "Construction of a Potential Live Aro Vaccine for typhoid type fever and cholorea- E. coli - related diarrheas," Infect. Immun., 46 : 564 - 9 (1984). Also see the other references cited in the above cited Brown, et al., article.

It is preferred to obtain the M protein gene from a virulent strain of S. pyogenes. However, it is possible to obtain the gene from an attenutated, non-virulent strain of S. pyogenes, or even to fabricate the nucleotide sequence coded for the desired M protein.

The recombinant DNA cloning vectors are not limited for use in a single species or strain of Salmonella. To the contrary, the vectors are broadly applicable and can be transformed in host cells of other gram negative bacteria such as of the Enterobacteriaceae genus (such as Shigella and Klebsiella like Klebsiella penumoniae, Enterobacter like Enterobacter aerogenes).Salmonellae , such as Salmonella arizona, and Citrobacter may be used if appropriately rendered non-virulent or attenuated.

Common Salmonella species which may be used when attenuated and rendered non-virulent include the following :

S. paratyphi A, S. schottmulleri, S typhimurium, S. paratyphi C, S. choleraesuis, S. montevideo, S. newport, S. typhi, S. enteritidis, S.gallinarum, S. anatum.

In accordance with the invention there may also be used as host for the recombinant DNA cloning vectors bacteria of the Streptococcus genus which are non-virulent or which have been made non-virulent or attenuated, including Streptococci of the immunological groups A-O but generally other than A. Suitable Streptococci which can be used as bacterial host include S. Cremoris, S. Faecalis, S. Salivarius, S. Mitior, S. Mitis, S. Mutans and S. Sanguis,the latter species is presently a preferred specie.

Additional appropriate microorganisms which may be attenuated and transformed in accordance with the invention are known. Reference may be made to Davis, et al., Microbiology (Harper & Row, Second Edition, 1973).

Generally any enteric bacterium may serve as the host bacterium. It is preferable that the host bacterium only survive in the subject long enough to elicit the opsonic response, but generally any bacterial strain that has been attenuated not to colonize but will still multiply to a limited degree to elicit antibodies to the foreign protein can be used. In a preferred embodiment of the invention the Aro⁻ strain of S. typhimurium is used, which requires two metabolites not found in mammalian tissues, PABA and 2,3-DHB. As a result, the innoculated bacteria die after several generations from lack of these metabolites. See Hoiseth and Stocker, cited above.

However, any mutated microbial agent with a metabolic deficiency for nutritional compounds not found in the tissues of the subject to be immunized, or one so made by genetic manipulations, may be employed.

It is to be noted that the non-virulent aro⁻ Salmonella typhimurium SL3261 into which a plasmid containing the structural gene encoding the serotype 5M protein antigen has been transformed expresses the entire M 5 protein molecule, which expression is confined almost exclusively to the S. typhimurium cytoplasmic compartment. It is a unique and unexpected aspect of this invention that an immunogenic and protective surface antigen such as the Streptococcal M protein antigen is expressed in the cytoplasm of the non-virulent host bacterium.

Thus it can be seen that the desired nucleotide sequence which codes for an expresses the protein antigen which is effective to elicit opsonic antibodies against streptococcal infections particularly of the serotype 5, can be cloned into a variety of hosts. In a broader sense therefore the transformed host in which the nucleotide sequence is found after replication need not be heterologous with respect to the nucleotide sequence, nor does the sequence need to be heterologous with respect to the microorganisms.

In accordance with a specific embodiment of the invention, it has been shown that a) peroral administration of up to $1,65 \times 10^9$ mutant non-virulent Salmonella containing the plasmid pMK207 encoding serotype 5 Streptococcal M protein was well tolerated in mice ; b) plasmid pMK207 was extremely stable both in vitro and in vivo ; c) the mice receiving the highest dose ($10^9$) of bacteria harbored the microorganisms in the liver for as long as three weeks without ill effects ; d) the mice immunized orally with non-virulent transformed Salmonella expressing the serotype 5 Streptococcal M protein antigen gene developed opsonic serum antibodies as early as three weeks against serotype M5 Streptococci ; and e) the immunized mice were completely protected at three weeks against intraperitoneal challenges of the homologous serotype m5 (but not the heterologous serotype M24) Streptococci. It is noteworthy that no cross-reactive immunity is observed when the composition of the invention is administered orally. The cytoplasmic expression of the M protein antigen in the non-virulent bacterium is especially advantageous for this oral administration. The antigen is protected within the cytoplasm of the non-virulent bacterium from the acids of the stomach and other damaging agents until the non-virulent cell dies and releases the antigen, ordinarily in the small intestine, the preferred location for delivery of the antigens.

The non-virulent bacterium may also be used as a host for recombinant DNA cloning vectors containing nucleotide sequences which code for and express immunogenic polypeptides which are specifically effective to confer immunity against Streptococcal infections and which are not cross-reactive with human tissue antigens, especially those of the heart.

Suitable immunogenic polypeptides include oligopeptides copying regions of M protein molecules lacking auto-immune epitopes, as described in United States Patent No. 4,284,537 issued to E. Beachey on August 18, 1981, United States Patent No. 4,454,121 issued to E. Beachey on June 12, 1984, United States Patent No. 4,521,334 issued to E. Beachey on June 4, 1985, United States Patent No. 4,597,967 issued to E. Beachey on July 1, 1986, United States Application Serial No. 739,963, to E. Beachey, et al, filed May 31, 1985, United States Application Serial No. 839,750 to E. Beachey et al filed March 14, 1986 and United States Application Serial No. 858,436 to E. Beachey et al filed May 1, 1986, all cited above.

To create a multivalent vaccine broadly protective against serotype M Streptococcus, one can use a M protein polypeptide sequence as a carrier to which the other sequences are covalently linked, evoking an opsonic response to each one of the Streptococcal serotypes, the polypeptides of which are linked to the carrier, as well as to the Streptococcal serotype, the polypeptide of which is serving as the carrier. This can be done by encoding a nucleotide sequence for such a multivalent polypeptide onto an S. pyogenes chromosome in the region of the M protein structureal gene, cloning it into plasmids and transforming the plasmids into novirulent host bacterium. Synthetic nucleotide sequences are preferred, but nucleotide sequences obtained from living bacterial cells may also be employed.

It is also possible to clone into non-virulent host bacterium already transformed to express immunogenic polypeptides eliciting opsonic responses to type M Streptococcal infections, genetic material for the expression of immunogenic polypeptides eliciting opsonic responses to other virulent bacteria species and the covalent linkage of both species polypeptides.

The resultant attenuated non-virulent host organism is orally administrable as a broad-sprectum vaccine according to the invention, capable of eliciting opsonic responses to more than one species of bacteria.

The capacity of serotype M 24 polypeptides as a carrier for a highly multi-valent vaccine is being tested with other serotype M polypeptide fragments.

Another approach of the invention has been to use Streptococcus sanguis bacteria which have been transformed with the M protein - expressing plasmids. The expression of serotype type 5 M protein in S. sanguis has been accomplished. The M protein gene was carried on a shuttle vector plasmid and transformed into the host bacterium, which was shown to express serotype 5 M protein fibrils on the surface of the organism. Moreover, the organism were able to bind fibrinogen which in turn rendered the microorganism resistant to phagocytosis.

A desirable method for immunizing against Streptococcal infections is to instill attenuated S. sanguis transformed in accordance with the invention, intranasally to evoke local immune response at the very site where Streptococci commonly enter the host.

Other advantages characteristic of the invention will appear from the non-limiting examples which follow.

EXAMPLE 1

TRANSFORMATION OF NON-VIRULENT HOST BACTERIUM TO EXPRESS STREPTOCOCCAL M PROTEIN.

The aro⁻ strain of Salmonella typhimurium SL3261, developed by Stocker and Hoseith (cited above) was chosen because this mutant form a microbial agent is capable of invasion but not causing disease. This mutant strain exhibits a nutritional marker for both p-aminobenzoic adid (PABA) and 2,3-dihydrobenzoic acid (DHB). See Stocker and Hoseith, cited above.

The M5 protein antigen structural gene (smp 5) was cloned and expressed in E. coli LE932 as described by Kehoe, et als., "Cloning and Genetic Analysis of Serotype 5M Protein Determinant of Group A Streptococci : Evidence for Puptiple Copies of the MS Determinant in the Streptococcus pyogenes Genome," Infect Immun., Vol. 48, pp. 190 - 197 (1985) and Poirer, et als., "Expression of Protective and Cardiac Tissue Cross-Reactive Epitopes of Type 5 Streptococcal M Protein in Escherichia coli," Infect Immun. Vol. 48, pp. 198 - 203 (1985), both of which are incorporated herein by reference.

The plasmid pMK207 was isolated, purified, and transformed first into an rm + strain LB5000 of Sal. typhimurium, as described by Bullas and Ryo, "Salmonella typhimurium LT2 Strains which are r⁻m + for all Three Chromosomally Located Systems of DNA Restriction and Modification," J. Bacteriol, Vol. 156, pp. 471 - 474 (1983) and Lederberg and Cohen, "Transformation of Salmonella typhimurium by Plasmid Deoxyribonucleic Acid," J. Bacteriol., Vol. 119, pp. 1072 - 1074 (1974), both of which are incorporated herein by reference. The plasmid isolated and purified from LB5000 was then used to transform the aro⁻Sal. typhimurium SL3261 in using the same above-cited procedures described by Lederberg and Cohen and Bullas and Ryo.

The transformed LB5000 and SL3261 expressed the entire M5 protein molecule as demonstrated by Western blot analysis of whole cell lysates (Fig. 1 ; lanes 1 and 2). The typical triplet of M5 protein (See Poirer, et als., and Kehoe, et als., cited above) migrated as bands of $M_r$ 59, 56 and 54k. Preliminary studies designed to determine the location of the M5 protein as expressed by Sal. typhimurium SL3261 indicated that the recombinant protein was confined almost exclusively to the cytoplasmic compartment. The M5 protein was expressed in a stable fashion by SL3261 even in the absence of antibiotic pressure, i.e. expression was apparent following repeated subcultures over five days, which represents approximatively 35 generations of growth (Fig. 1 ; lanes 3 - 8).

EXAMPLE 2

MOUSE TOLERANCE OF TRANSFORMED SAL. TYPHIMURIUM SL3261

In preliminary studies, BALB/c mice were challenged with increasing doses of SL3261-pMK207 to determine whether or not the animals would tolerate the transformed organisms. None of the mice receiving up to a maximum of $1,65 \times 10^9$ organisms per oral dose either sickened or died. At 1, 3, 5 and 10 weeks after innoculation, two mice from each dosage group were sacrificed and their livers, spleens and intestines cultured for SL3261-pMK207 on McConkey's agar containing 50 mg/ml kanamycin sulfate and 10 mg/ml each of PABA and DHB. Non-lactose fermenting colonies could be isolated at one week only from the mice receiving $10^6$ organisms or more, and at three weeks, only from those receiving $10^9$ organisms (Table 1). No isolates were recovered after three weeks. The colonies isolated at three weeks expressed an intact M5 protein, which suggests that smp5 was stable in vivo (Fig. 1 ; lane 9). The 1, 3,5, 9, 10, 15 and 20 weeks sera from mice innoculated orally were shown to exhibit antibodies against type 5M protein and type 5 Streptococci by ELISA and opsonization experiments, respectively (Table 2). The saliva obtained from mice

7

that were 20 weeks post-immunization was also shown to possess anti-M5 protein antibodies, primarily of the IgA class (Table 3).

TABLE 1

DISTRIBUTION OF SALMONELLA TYPHIMURIUM SL3261 IN

THE ORGANS OF BALB/c MICE AFTER ORAL IMMUNIZATION

| INOCULATION DOSE[1] | | SALMONELLA DISTRIBUTION IN ORGANS[2]: | | | | | | | | |
| Initial | Boost | INTESTINE | | | LIVER - GALL BLADDER | | | SPLEEN | | |
| | | Week 1 | Week 2 | Week 3 | Week 1 | Week 2 | Week 3 | Week 1 | Week 2 | Week 3 |
| $1.7 \times 10^9$ | $1.1 \times 10^9$ | $2.1 \times 10^4$ | <50 | <50 | $4 \times 10^2$ | $3 \times 10^2$ | <50 | $4 \times 10^2$ | <50 | <50 |
| $1.7 \times 10^8$ | $1.1 \times 10^8$ | <200 | <50 | ND[3] | $8 \times 10^2$ | <50 | ND | $4 \times 10^2$ | <50 | ND |
| $1.7 \times 10^7$ | $1.1 \times 10^7$ | <200 | <50 | ND | $1 \times 10^2$ | <50 | ND | $1 \times 10^2$ | <50 | ND |
| $1.7 \times 10^6$ | $1.1 \times 10^6$ | <100 | ND | ND | <50 | ND | ND | <50 | ND | ND |
| $1.7 \times 10^5$ | $1.1 \times 10^5$ | <100 | ND | ND | <50 | ND | ND | <50 | ND | ND |
| $1.7 \times 10^4$ | $1.1 \times 10^4$ | <100 | ND | ND | <50 | ND | ND | <50 | ND | ND |

1. Mice were given serially 10-fold diluted oral doses of *S.typhimurium* strain SL3261 begining with $1.7 \times 10^9$ colony forming units (cfu) on day 1 (initial dose), with an oral booster dose begining with $1.1 \times 10^9$ cfu on day 5. Each dose was suspended in 25 µl of phosphate buffered saline (PBS, pH 7.2) containing 5 µg ml$^{-1}$ kanamycin sulfate, and 1 µg ml$^{-1}$ each of paraminobenzoic acid (PABA) and 2,3-dihydroxybenzoic acid (DHB). Mice were sacrificed at 1, 2, and 3 weeks after administration of the initial dose.

2. Intestine (pyloric sphincter to rectum), liver-gall bladder, and spleen were removed aseptically, placed in PBS, homogenized with a Cellector™ (100 mesh; Bellco), and plated onto MacConkey agar containing 10 µg ml$^{-1}$ each of PABA and DHB with or without 50 µg ml$^{-1}$ of kanamycin.

3. Not determined.

TABLE 2

| Opsonization of M Type 5 *S.pyogenes* with Mouse Anti-M5-Protein-Serum Obtained from BALB/c Mice Orally Immunized with *S.typhimurium* SL3261-pMK207 and their Anti-pepM Protein ELISA Titers. | | | | | |
|---|---|---|---|---|---|
| Test Serum | ELISA Titer[1] | | | Percent Opsonization[2] | |
| | IgA | IgG | IgM | Type 5 | Type 24 |
| Pre-immune | <50 | <50 | <50 | 0 | 2 |
| 1 week | 100 | 200 | <50 | 4 | 4 |
| 3 weeks | 400 | 400 | <50 | 36 | 0 |
| 5 weeks | 800 | 800 | <50 | 52 | 2 |
| 9 weeks | 100 | 1600 | 200 | 90 | 2 |
| 10 weeks | 50 | 800 | 400 | 92 | 2 |
| 15 weeks | 100 | 800 | 400 | 52 | 0 |
| 15 weeks[3] | 800 | 12800 | 800 | 82 | 0 |
| 20 weeks | <50 | 400 | 200 | 10 | 2 |
| 20 weeks[3] | 400 | 3200 | 200 | 80 | 2 |
| Anti-pepM5[4] | ND[5] | ND | ND | 96 | 2 |
| Anti-pepM24[4] | ND | ND | ND | 0 | 98 |

1. ELISA titers were determined using immobilized pepM5 reacted with BALB/c mouse anti-SL3261-pMK207-sera followed with peroxidase conjugated goat anti-mouse -IgA, -IgG, or -IgM. All mouse antisera reacted against immobilized pepM24 gave titers of <50.

2. Opsonization was determined as the percent of total neutrophils with associated streptococci.

3. Mice were boosted 48 hours prior to bleed out with 50 $\mu$g of pepM5 in 0.1 ml of phosphate buffered saline, pH 7.2.

4. Both anti-pepM5 and anti-pepM24 were prepared in rabbits to serve as positive (homologous reaction) and negative (heterologous reaction) controls.

5. The antibody titers for the rabbit anti-pepM5 or anti-pepM24 were not determined.


TABLE 3

| Salivary Antibody Response[1] Against M5-Protein BALB/c Mice Immunized Orally with *S.typhimurium* SL3261-pMK207. | | |
|---|---|---|
| Test Serum | ELISA Titer[2] Against pepM5 | |
| | IgA | IgA + IgG + IgM |
| Pre-immune | <4 | <4 |
| 20 Weeks[3] | 32 | 32 |
| 20 Weeks + Boost[4] | 64 | 128 |

1. Saliva was collected and pooled from mice (four per group) following induction intraperitoneally with 0.1 ml of 2% pilocarpine.

2. ELISA titers were determined using immobilized pepM5 reacted with saliva (serially diluted two-fold) collected from BALB/c mice immunized with SL3261-pMK207 followed with peroxidase conjugated goat anti-mouse -IgA or an anti-mouse - IgA + IgG + IgM mixture.

3. Mice were immunized 20 weeks prior to the saliva collection (see Table 2).

4. Mice were injected with booster dose of 50 $\mu$g of pepM5 in 0.1 ml of phosphate buffered saline, pH 7.2, 60 hours prior to saliva collection.


EXAMPLE 3

A group of mice was innoculated orally with two doses of SL3261-pMK207 and challenged 22 days after the first dose with type 5 or 24 Streptococci ; or the virulent parent Sal. typhimurium 1344 (Table 3).

As can readily be seen by the results, the mice receiving the M5 expressing Sal. typhimurium mutant were completely protected against an intra-peritoneal challenge of type 5 Streptococci, but not with type 24 Streptococci. Control mice challenged intra-peritoneally with the virulent SL1344 Sal. typhimurium were also protected. The type 5 challenged mice were protected against a dose that exceeded the $LD_{50}$ by 100-fold (Table 4). This protection against parenteral challenge by type 5 Streptococci indicates that the immunity conferred is systemic.

TABLE 4

| Challenge by Intra-Pentoneal Injection of M Type 5 and Type 24 Streptococci or $S.typhimurium$ SL1344 in BALB/c Mice Immunized Orally[1] with Live aro$^{-}$ $S.typhimurium$ Transformed with pMK207 Expressing Type 5 M Protein. | | | |
|---|---|---|---|
| Challenge Organisms[2] | Dose[3] | Survival[4,5] | |
| | | Unimmunized | Immunized |
| M5 Streptococci (Smith strain) | $1.7 \times 10^4$ | 2/4 | 4/5 |
| | $1.7 \times 10^5$ | 0/4 | 5/5 |
| | $1.7 \times 10^6$ | 0/4 | 5/5 |
| M24 Streptococci (Vaughn strain) | $1.6 \times 10^3$ | 1/3 | 1/3 |
| | $1.6 \times 10^4$ | 0/3 | 0/3 |
| | $1.6 \times 10^5$ | 0/3 | 0/3 |
| $S.typhimurium$ (Strain SL1344) | $7.3 \times 10^1$ | 0/3 | 3/3 |
| | $7.3 \times 10^2$ | 0/3 | 3/3 |
| | $7.3 \times 10^3$ | 0/3 | 3/3 |

1. Each immunized mouse received $1.2 \times 10^9$ pMK207-transformed SL3261 $S.typhimurium$ colony forming units (cfu) at 22 days and $1.6 \times 10^9$ cfu at 17 days before challenged. Each dose was administered orally suspended in 25 $\mu$l of phosphate buffered saline containing 5 $\mu$g ml$^{-1}$ kanamycin sulfate, and 1 $\mu$g ml$^{-1}$ each of paraminobenzoic acid and 2,3-dihydroxybenzoic acid.
2. The $LD_{50}$ for M5 and M24 streptococci, and SL1344 in BALB/c mice was approximately $2 \times 10^4$, $3 \times 10^3$, and $1 \times 10^3$ CFU, respectively.
3. Dose was determined as the CFU administered.
4. Survival was recorded as the number of surviving mice divided by the number of mice challenged.
5. All recorded deaths occurred between 3 and 6 days after challenged. All surviving mice appeared healthy up to 30 days after challenge.

A group of BALB/c mice which had been immunized orally with SL3261-pMK207 were challenged intra-nasally with type 5 or 24 Streptococci, or the virulent parent Sal. typhimurium SL1344. As can be seen in Table 5, only those mice immunized orally with SL3261-pMK207 were able to survive an intra-nasal challenge with a dosage of homologous organisms which was otherwise sufficient to kill all immunized animals. Further, the protection conferred by Sal. typhimurium SL3261 expressing recombinant M5 protein is M type specific as demonstrated by the inability of intra-nasally challenged mice to tolerate M type 24 Streptococci. The fact that mice were refractory to challenge by intra-nasal innoculation suggested that oral immunization with M5 protein expressing Sal. typhimurium SL3261 was sufficient to inpart local immunity.

EP 0 305 279 B1

TABLE 5

| Challenge by Intra-Nasal Inoculation of M Type 5 and Type 24 Streptococci or *S.typhimurium* SL1344 in BALB/c Mice Immunized Orally[1] with Live aro⁻ *S.typhimurium* Transformed with pMK207 Expressing Type 5 M Protein. | | | |
|---|---|---|---|
| Challenge Organisms[2] | Dose[3] | Survival[4,5] | |
| | | Unimmunized | Immunized |
| M5 Streptococci (Smith strain) | $3.9 \times 10^7$ | 0/4 | 6/6 |
| M24 Streptococci (Vaughn strain) | $3.5 \times 10^7$ | 0/4 | 0/6 |
| *S.typhimurium* (Strain SL1344) | $4.3 \times 10^4$ | 0/4 | 6/6 |

1. Each immunized mouse received $1.2 \times 10^9$ pMK207 transformed SL3261 *S.typhimurium* colony forming units (cfu) at day 1 and $1.6 \times 10^9$ cfu at day 5. Each dose was administered orally suspended in 25 $\mu$l of phosphate buffered saline (PBS, pH 7.2) containing 5 $\mu$g ml⁻¹ kanamycin sulfate, and 1 $\mu$g ml⁻¹ each of paraminobenzoic acid and 2,3-dihydroxybenzoic acid.
2. Challenge dose was determined as the cfu administered intra-nasally in 20 $\mu$l of PBS (10 $\mu$l per nostril).
3. Mice were challenged 13 weeks after the initial immunization (i.e. day 1).
4. Survival was recorded as the number of surviving mice over the number of mice challenged.
5. Deaths from streptococci occurred between 2 and 4 days, while deaths due the salmonella occurred between 5 and 7 days after challenge. All surviving mice appeared healthy up to 30 days after challenge.

The above examples are not to be construed as limitations, but are merely illustrative of the invention.

**Claims**
**Claims for the following Contracting States : AT, BE, CH, DE, FR, GB, IT, LI, LU, NL, SE**

1. A non-virulent live bacterium transformed by an heterologous nucleotide sequence expressing at least a serotype-specific streptococcal M protein immunizing antigen which is effective to elicit opsonic antibodies against streptococcal infections without eliciting antibodies which are cross-reactive with human heart tissue antigens, for use as a vaccine.

2. The bacterium for use as a vaccine of claim 1, wherein the heterologous nucleotide sequence is contained in a plasmid.

3. The bacterium for use as a vaccine of any of claims 1 or 2, wherein the heterologous nucleotide sequence expresses at least an antigen selected from the group consisting of M protein antigens effective to elicit opsonic antibodies against Streptococci of serotypes 5, 6 and 24.

4. The bacterium for use as a vaccine of claim 3, wherein the heterologous nucleotide sequence expresses at least a streptococcal M protein antigen which confers immunity against the type 5 Streptococci.

5. A non-virulent live bacterium transformed by an heterologous nucleotide sequence expressing at least a serotype-specific streptocccocal M Protein immunizing antigen effective to elicit opsonic antibodies againts streptococcal infections without eliciting antibodies which are cross reactive with human heart tissue antigens, wherein said bacterium has a mutational variation causing a nutritional deficiency.

6. A non-virulent live bacterium transformed by an heterologous nucleotide sequence expressing at least a streptococcal M protein immunizing antigen which is effective to elicit opsonic antibodies against streptococcal infections without eliciting antibodies which are cross-reactive with human heart tissue antigens, wherein said bacterium is selected from the group consisting of <u>Salmonella</u> and <u>Streptococ-</u>

11

cus.

7. The bacterium of claim 6, which is a transformed non-virulent Salmonella selected from the group consisting of : Salmonella paratyphi, Salmonella schottmulleri, Salmonella typhimurium, Salmonella choleraesuis, Salmonella montevideo, Salmonella newport, Salmonella typhi, Salmonella enteritidis, Salmonella gallinarum, and Salmonella anatum.

8. The bacterium of claim 7, which is a transformed non-virulent strain of Salmonella typhimurium.

9. The bacterium of claim 6, which is a transformed non-virulent Streptococcus selected from the group consisting of : Streptococcus cremoris, Streptococcus faecalis, Streptococcus salivarius, Streptococcus mitior, Streptococcus mitis, Streptococcus mutans and Streptococcus sanguis.

10. The bacterium of claim 9, which is a transformed non-virulent strain of Streptococcus sanguis, or of Streptococcus mutans.

11. The bacterium of any of claims 5 to 10, wherein the heterologous nucleotide sequence expresses at least an antigen selected from the group consisting of M protein antigens effective to elicit opsonic antibodies against Streptococci of types 5, 6 and 24.

12. The bacterium of claim 11, wherein the heterologous nucleotide sequence expresses at least a streptococcal M protein antigen which confers immunity against Streptococci of type 5 .

13. The bacterium of any of claims 5 to 12, for use as a vaccine.

14. The bacterium for use as a vaccine of any of claims 1 to 4 or 13, wherein said vaccine confers systemic immunity.

15. The bacterium for use as a vaccine of any of claims 1 to 4 or 13, wherein said vaccine confers local immunity.

16. The bacterium for use as a vaccine of any of claims 1 to 4 or 13 to 15, wherein said vaccine confers immunity through oral administration.

17. A therapeutic composition which comprises a biologically acceptable carrier and a non virulent, live transformed bacterium as defined in any of claims 1 to 16.

18. The therapeutic composition of claim 17, wherein said composition is orally administrable.

**Claims for the following Contracting State : ES**

1. A process for preparing a vaccine wherein said process comprises the incorporation, as the active vaccinating agent, of a non-virulent live bacterium transformed by an heterologous nucleotide sequence expressing at least a serotype-specific streptococcal M protein immunizing antigen, which is effective to elicit opsonic antibodies against streptococcal infections without eliciting antibodies which are cross-reactive with human heart tissue antigens.

2. The process of claim 1, wherein the heterologous nucleotide sequence is contained in a plasmid.

3. The process of any of claims 1 or 2, wherein the heterologous nucleotide sequence expresses at least an antigen selected from the group consisting of M protein antigens effective to elicit opsonic antibodies against Streptococci of serotypes 5, 6 and 24.

4. The process of claim 3, wherein the heterologous nucleotide sequence expresses at least a streptococcal M protein antigen which confers immunity against the type 5 Streptococci.

5. A process for preparing a non-virulent transformed live bacterium, by transforming a non-virulent bacterium with an heterologous nucleotide sequence expressing at least a serotype-specific streptoccoccal M Protein immunizing antigen, effective to elicit opsonic antibodies against streptococcal infections without eliciting antibodies which are cross-reactive with human heart tissue antigens, wherein said bacterium has a mutational variation causing a nutritional deficiency.

6. A process for preparing a non-virulent transformed live bacterium by transformation of a non-virulent bacterium with an heterologous nucleotide sequence expressing at least a streptococcal M protein immunizing antigen which is effective to elicit opsonic antibodies against streptococcal infections without eliciting antibodies which are cross-reactive with human heart tissue antigens, wherein said non-virulent host bacterium is selected from the group consisting of Salmonella and Streptococcus.

7. The process of claim 6, wherein the non-virulent transformed host is a Salmonella selected from the group consisting of : Salmonella paratyphi, Salmonella schottmulleri, Salmonella typhimurium, Salmonella choleraesuis, Salmonella montevideo, Salmonella newport, Salmonella typhi, Salmonella enteritidis, Salmonella gallinarum, and Salmonella anatum.

8. The process of claim 7, wherein the Salmonella is a non-virulent transformed strain of Salmonella typhimurium.

9. The process of claim 6, wherein the non-virulent transformed host is a Streptococcus selected from the group consisting of : Streptococcus cremoris, Streptococcus faecalis, Streptococcus salivarius, Streptococcus mitior, Streptococcus mitis, Streptococcus mutans and Streptococcus sanguis.

10. The process of claim 9, wherein the non-virulent transformed host is a strain of Streptococcus sanguis, or of Streptococcus mutans.

11. The process of any of claims 5 to 10, wherein the heterologous nucleotide sequence expresses at least an antigen selected from the group consisting of M protein antigens effective to elicit opsonic antibodies against Streptococci of types 5, 6 and 24.

12. The process of claim 11, wherein the heterologous nucleotide sequence expresses a streptococcal M protein antigen which confers immunity against Streptococci of type 5 .

13. A process for preparing a vaccine wherein said process comprises the incorporation as active vaccinating agent of a bacterium obtained by a process according to any of claims 5 to 12.

14. The process of any of claims 1 to 4 or 13, wherein said vaccine confers systemic immunity.

15. The process of any of claims 1 to 4 or 13, therein said vaccine confers local immunity.

16. The process of any of claims 1 to 4 or 13 to 15, wherein said vaccine confers immunity through oral administration.

17. A process for preparing a therapeutic composition which comprises mixing a biologically acceptable carrier and a non virulent, live transformed bacterium as defined in any of claims 1 to 16.

18. The process of claim 17, wherein said composition is orally administrable.

**Patentansprüche**
**Patentansprüche für folgende Vertragsstaaten : AT, BE, CH, DE, FR, GB, IT, LI, LU, NL, SE**

1. Nicht-virulentes lebendes Bakterium, transformiert durch eine heterologe Nucleotidsequenz, die mindestens ein Serotyp-spezifisches immunisierendes Streptococcen-M-Protein-Antigen exprimiert, welches das Hervorbringen von opsonisierenden Antikörpern gegen Streptococcen-Infektionen bewirkt, ohne Antikörper hervorzubringen, die mit menschlichen Herzgewebs-Antigenen kreuzreagieren, zur Verwendung als Impfstoff.

2. Bakterium zur Verwendung als Impfstoff nach Anspruch 1, dadurch **gekennzeichnet**, daß die heterologe Nucleotidsequenz in einem Plasmid enthalten ist.

**3.** Bakterium zur Verwendung als Impfstoff nach einem der Ansprüche 1 oder 2, dadurch **gekennzeichnet**, daß die heterologe Nucleotidsequenz mindestens ein Antigen, ausgewählt aus der Gruppe bestehend aus M-Protein-Antigenen, die das Hervorbringen von opsonisierenden Antikörpern gegen Streptococcen der Serotypen 5, 6 und 24 bewirken, exprimiert.

**4.** Bakterium zur Verwendung als Impfstoff nach Anspruch 3, dadurch **gekennzeichnet**, daß die heterologe Nucleotidsequenz mindestens ein Streptococcen-M-Protein-Antigen exprimiert, das Immunität gegen Typ 5-Streptococcen verleiht.

**5.** Nicht-virulentes lebendes Bakterium, transformiert durch eine heterologe Nucleotidsequenz, die mindestens ein Serotyp-spezifisches immunisierendes Streptococcen-M-Protein-Antigen exprimiert, welches das Hervorbringen von opsonisierenden Antikörpern gegen Streptococcen-Infektionen bewirkt, ohne Antikörper hervorzubringen, die mit menschlichen Herzgewebs-Antigenen kreuzreagieren, wobei das Bakterium eine Mutationsveränderung aufweist, die einen ernährungsbedingten Mangel verursacht.

**6.** Nicht-virulentes lebendes Bakterium, transformiert durch eine heterologe Nucleotidsequenz, welche mindestens ein immunisierendes Streptococcen-M-Protein-Antigen, welches das Hervorbringen von opsonisierenden Antikörpern gegen Streptococcen-Infektionen bewirkt, ohne Antikörper hervorzubringen, die mit menschlichen Herzgewebs-Antigenen kreuzreagieren, exprimiert, wobei das Bakterium aus der Gruppe bestehend aus Salmonella und Streptococcus ausgewählt ist.

**7.** Bakterium nach Anspruch 6, dadurch **gekennzeichnet**, daß es sich um eine transformierte nicht-virulente Salmonella handelt, ausgewählt aus der Gruppe bestehend aus Salmonella paratyphi, Salmonella schottmulleri, Salmonella typhimurium, Salmonella choleraesuis, Salmonella montevideo, Salmonella newport, Salmonella typhi, Salmonella enteritidis, Salmonella gallinarum und Salmonella anatum.

**8.** Bakterium nach Anspruch 7, dadurch **gekennzeichnet**, daß es sich um einen transformierten nicht-virulenten Stamm von Salmonella typhimurium handelt.

**9.** Bakterium nach Anspruch 6, dadurch **gekennzeichnet**, daß es sich um einen transformierten nicht-virulenten Streptococcus handelt, ausgewählt aus der Gruppe bestehend aus Streptococcus cremoris, Streptococcus faecalis, Streptococcus salivarius, Streptococcus mitior, Streptococcus mitis, Streptococcus mutans und Streptococcus sanguis.

**10.** Bakterium nach Anspruch 9, dadurch **gekennzeichnet**, daß es sich um einen transformierten nicht-virulenten Stamm von Streptococcus sanguis oder Streptococcus mutans handelt.

**11.** Bakterium nach einem der Ansprüche 5 bis 10, dadurch **gekennzeichnet**, daß die heterologe Nucleotidsequenz mindestens ein Antigen, ausgewählt aus der Gruppe bestehend aus M-Protein-Antigenen, die das Hervorbringen von opsonisierenden Antikörpern gegen Streptococcen der Typen 5, 6 und 24 bewirken, exprimiert.

**12.** Bakterium nach Anspruch 11, dadurch **gekennzeichnet**, daß die heterologe Nucleotidsequenz mindestens ein Streptococcen-M-Protein-Antigen exprimiert, das Immunität gegen Streptococcen vom Typ 5 verleiht.

**13.** Bakterium nach einem der Ansprüche 5 bis 12 zur Verwendung als Impfstoff.

**14.** Bakterium zur Verwendung als Impfstoff nach einem der Ansprüche 1 bis 4 oder 13, dadurch **gekennzeichnet**, daß der Impfstoff systemische Immunität verleiht.

**15.** Bakterium zur Verwendung als Impfstoff nach einem der Ansprüche 1 bis 4 oder 13, dadurch **gekennzeichnet**, daß der Impfstoff Gewebsimmunität verleiht.

**16.** Bakterium zur Verwendung als Impfstoff nach einem der Ansprüche 1 bis 4 oder 13 bis 15, dadurch **gekennzeichnet**, daß der Impfstoff die Immunität durch orale Verabreichung verleiht.

**17.** Therapeutisches Präparat, welches einen biologisch annehmbaren Träger und ein nicht-virulentes, lebendes transformiertes Bakterium, wie in einem der Ansprüche 1 bis 16 definiert, umfaßt.

**18.** Therapeutisches Präparat nach Anspruch 17, dadurch **gekennzeichnet**, daß das Präparat oral verabreicht werden kann.

**Patentansprüche für folgenden Vertragsstaat : ES**

**1.** Verfahren zur Herstellung eines Impfstoffs, dadurch **gekennzeichnet**, daß das Verfahren das Einbringen als aktives Impfmittel eines nicht-virulenten lebenden Bakteriums, transformiert durch eine heterologe Nucleotidsequenz, die mindestens ein immunisierendes Serotyp-spezifisches Streptococcen-M-Protein-Antigen, welches das Hervorbringen von opsonisierenden Antikörpern gegen Streptococcen-Infektionen bewirkt, ohne Antikörper hervorzubringen, die mit menschlichen Herzgewebs-Antigenen kreuzreagieren, exprimiert, umfaßt.

**2.** Verfahren nach Anspruch 1, dadurch **gekennzeichnet**, daß die heterologe Nucleotidsequenz in einem Plasmid enthalten ist.

**3.** Verfahren nach einem der Ansprüche 1 oder 2, dadurch **gekennzeichnet**, daß die heterologe Nucleotidsequenz mindestens ein Antigen, ausgewählt aus der Gruppe bestehend aus M-Protein-Antigenen, die das Hervorbringen von opsonisierenden Antikörpern gegen Streptococcen der Serotypen 5, 6 und 24 bewirken, exprimiert.

**4.** Verfahren nach Anspruch 3, dadurch **gekennzeichnet**, daß die heterologe Nucleotidsequenz mindestens ein Streptococcen-M-Protein-Antigen, das Immunität gegenüber Typ 5-Streptococcen verleiht, exprimiert.

**5.** Verfahren zur Herstellung eines nicht-virulenten, transformierten lebenden Bakteriums durch Transformieren eines nicht-virulenten Bakteriums mit einer heterologen Nucleotidsequenz, die mindestens ein immunisierendes Serotyp-spezifisches Streptococcen-M-Protein-Antigen, welches für das Hervorbringen von opsonisierenden Antikörpern gegen Streptococcen-Infektionen wirksam ist, ohne Antikörper hervorzubringen, die mit menschlichen Herzgewebs-Antigenen kreuzreagieren, exprimiert, wobei das Bakterium eine Mutationsveränderung aufweist, die einen ernährungsbedingten Mangel verursacht.

**6.** Verfahren zur Herstellung eines nicht-virulenten, transformierten lebenden Bakteriums durch Transformation eines nicht-virulenten Bakteriums mit einer heterologen Nucleotidsequenz, die mindestens ein immunisierendes Streptococcen-M-Protein-Antigen, welches das Hervorbringen von opsonisierenden Antikörpern gegen Streptococcen-Infektionen bewirkt, ohne Antikörper hervorzubringen, die mit menschlichen Herzgewebs-Antigenen kreuzreagieren, exprimiert, wobei das nicht-virulente Wirtsbakterium ausgewählt wird aus der Gruppe bestehend aus Salmonella und Streptococcus.

**7.** Verfahren nach Anspruch 6, dadurch **gekennzeichnet**, daß es sich bei dem nicht-virulenten transformierten Wirt um eine Salmonella handelt, ausgewählt aus der Gruppe bestehend aus Salmonella paratyphi, Salmonella schottmulleri, Salmonella typhimurium, Salmonella choleraesuis, Salmonella montevideo, Salmonella newport, Salmonella typhi, Salmonella enteritidis, Salmonella gallinarum und Salmonella anatum.

**8.** Verfahren nach Anspruch 7, dadurch **gekennzeichnet**, daß es sich bei der Salmonella um einen nicht-virulenten transformierten Stamm von Salmonella typhimurium handelt.

**9.** Verfahren nach Anspruch 6, dadurch **gekennzeichnet**, daß es sich bei dem nicht-virulenten transformierten Wirt um einen Streptococcus, ausgewählt aus der Gruppe bestehend aus Streptococcus cremoris, Streptococcus faecalis, Streptococcus salivarius, Streptococcus mitior, Streptococcus mitis, Streptococcus mutans und Streptococcus sanguis handelt.

**10.** Verfahren nach Anspruch 9, dadurch **gekennzeichnet**, daß der nicht-virulente transformierte Wirt ein Stamm von Streptococcus sanguis oder von Streptococcus mutans ist.

15

**11.** Verfahren nach einem der Ansprüche 5 bis 10, dadurch **gekennzeichnet**, daß die heterologe Nucleotidsequenz mindestens ein Antigen, ausgewählt aus der Gruppe bestehend aus M-Protein-Antigenen, die das Hervorbringen von opsonisierenden Antikörpern gegen Streptococcen der Typen 5, 6 und 24 bewirken, exprimiert.

**12.** Verfahren nach Anspruch 11, dadurch **gekennzeichnet**, daß die heterologe Nucleotidsequenz ein Streptococcen-M-Protein-Antigen exprimiert, das Immunität gegenüber Streptococcen vom Typ 5 verleiht.

**13.** Verfahren zur Herstellung eines Impfstoffs, dadurch **gekennzeichnet**, daß das Verfahren das Einbringen eines aktiven Impfmittels eines Bakteriums, erhalten nach einem Verfahren gemäß einem der Ansprüche 5 bis 12, umfaßt.

**14.** Verfahren nach einem der Ansprüche 1 bis 4 oder 13, dadurch **gekennzeichnet**, daß der Impfstoff systemische Immunität verleiht.

**15.** Verfahren nach einem der Ansprüche 1 bis 4 oder 13, dadurch **gekennzeichnet**, daß der Impfstoff Gewebsimmunität verleiht.

**16.** Verfahren nach einem der Ansprüche 1 bis 4 oder 13 bis 15, dadurch **gekennzeichnet**, daß der Impfstoff Immunität durch orale Verabreichung verleiht.

**17.** Verfahren zur Herstellung eines therapeutischen Präparats, dadurch **gekennzeichnet**, daß man einen biologisch annehmbaren Träger und ein nicht-virulentes, lebendes transformiertes Bakterium, wie in einem der Ansprüche 1 bis 16 definiert, vermischt.

**18.** Verfahren nach Anspruch 17, dadurch **gekennzeichnet**, daß das Präparat oral verabreicht werden kann.

**Revendications**
**Revendications pour les Etats contractants suivants : AT, BE, CH, DE, FR, GB, IT, LI, LU, NL, SE**

**1.** Bactérie vivante non virulente, transformée par une séquence de nucléotides hétérologue exprimant au moins un antigène immunisant de protéine M streptoccique spécifique d'un sérotype qui est efficace pour provoquer des anticorps opsoniques contre des infections streptocciques sans générer d'anticorps qui présentent une réaction croisée avec des antigènes de tissus cardiaque humain pour l'utilisation en tant que vaccin.

**2.** Bactérie pour l'utilisation comme vaccin suivant la revendication 1, caractérisée en ce que la séquence de nucléotides hétérologue est contenue dans un plasmide.

**3.** Bactérie pour l'utilisation comme vaccin suivant les revendications 1 ou 2, caractérisée en ce que la séquence de nucléotides hétérologue exprime au moins un antigène choisi dans le groupe consistant en antigènes de protéine M efficaces pour générer des anticorps opsoniques contre des streptocoques des sérotypes 5, 6 et 24.

**4.** Bactérie pour l'utilisation comme vaccin suivant la revendication 3, caractérisée en ce que la séquence de nucléotides hétérologue exprime au moins un antigène de protéine M streptococcique qui confère un immunité contre les streptocoques de type 5.

**5.** Bactérie vivante non virulente transformée par une séquence de nucléotides hétérologue exprimant au moins un antigène immunisant de protéine M streptoccique spécifique d'un sérotype efficace pour générer des anticorps opsoniques contre des infections streptocciques sans générer d'anticorps qui présentent une réaction croisée avec des antigènes de tissu cardiaque humain, caractérisée en ce que cette bactérie a une variation mutationnelle provoquant une déficience nutritionnelle.

**6.** Bactérie vivante non virulente transformée par une séquence de nucléotides hétérologue exprimant au moins un antigène immunisant de protéine M streptoccique qui est efficace pour générer des anticorps

16

opsoniques contre des infections streptocciques sans générer d'anticorps qui présentent une réaction croisée avec des antigènes de tissu cardiaque humain, caractérisée en ce que cette bactérie est choisie dans le groupe consistant en Salmonella et Streptococcus.

7. Bactérie suivant la revendication 6, qui est une *Salmonella* non virulente transformée choisie dans le groupe consistant en *Salmonella paratyphi*, *Salmonella schottmulleri*, *Salmonella typhimurium*, *Salmonella choleraesuis*, *Salmonella montevideo*, *Salmonella newport*, *Salmonella typhim*, *Salmonella enteritidis*, *Salmonella gallinarum* et *Salmonella anatum*.

8. Bactérie suivant la revendication 7, qui est une souche non virulente transformée de *Salmonella typhimurium*.

9. Bactérie suivant la revendication 6, qui est un *Streptococcus* non virulent transformé choisi dans le groupe consistant en *Streptococcus cremoris*, *Streptococcus faecalis*, *Streptococcus salivarius*, *Streptococcus mitior*, *Streptococcus mitis*, *Streptococcus mutans* et *Streptococcus sanguis*.

10. Bactérie suivant la revendication 9, qui est une souche non virulente transformée de *Streptococcus sanguis* ou de *Streptococcus mutans*.

11. Bactérie suivant l'une quelconque des revendications 5 à 10, caractérisée en ce que la séquence de nucléotides hétérologue exprime au moins un antigène choisi dans le groupe consistant en antigènes de protéine M efficaces pour générer des anticorps opsoniques contre des streptocoques de types 5, 6 et 24.

12. Bactérie suivant la revendication 11, caractérisée en ce que la séquence de nucléotides hétérologue exprime au moins un antigène de protéine M streptococcique qui confère une immunité contre les streptocoques de type 5.

13. Bactérie suivant l'une quelconque des revendications 5 à 12, pour l'utilisation comme vaccin.

14. Bactérie pour l'utilisation comme vaccin suivant l'une quelconque des revendications 1 à 4 ou 13, caractérisée en ce que ce vaccin confère une immunité systématique.

15. Bactérie pour l'utilisation comme vaccin suivant l'une quelconque des revendications 1 à 4 ou 13, caractérisée en ce que ce vaccin confère une immunité locale.

16. Bactérie pour l'utilisation comme vaccin suivant l'une quelconque des revendications 1 à 4 ou 13 à 15, caractérisée en ce que ce vaccin confère une immunité par administration orale.

17. Composition thérapeutique qui comprend un support acceptable du point de vue biologique et une bactérie transformée vivante non virulente suivant l'une quelconque des revendications 1 à 16.

18. Composition thérapeutique suivant la revendication 17, qui peut être administrée par voie orale.

**Revendications pour l'Etat contractant suivant : ES**

1. Procédé pour la préparation d'un vaccin, qui comprend l'incorporation, en tant qu'agent vaccinant actif, d'une bactérie vivante non virulente transformée par une séquence de nucléotides hétérologue exprimant au moins un antigène immunisant de protéine M streptococcique spécifique d'un sérotype qui est efficace pour provoquer des anticorps opsoniques contre des infections streptocciques sans générer d'anticorps qui présentent une réaction croisée avec des antigènes de tissu cardiaque humain.

2. Procédé suivant la revendication 1, dans lequel la séquence de nucléotides hétérologue est contenue dans un plasmide.

3. Procédé suivant les revendications 1 ou 2, dans lequel la séquence de nucléotides hétérologue exprime au moins un antigène choisi dans le groupe consistant en antigènes de protéine M efficaces pour générer des anticorps opsoniques contre des streptocoques des sérotypes 5, 6 et 24.

**4.** Procédé suivant la revendication 3, dans lequel la séquence de nucléotides hétérologue exprime au moins un antigène de protéine M streptococcique qui confère une immunité contre les streptocoques de type 5.

**5.** Procédé pour la préparation d'une bactérie vivante transformée non virulente, par transformation d'une bactérie non virulente avec une séquence de nucléotides hétérologue exprimant au moins un antigène immunisant de protéine M streptoccique spécifique d'un sérotype efficace pour générer des anticorps opsoniques contre des infections streptocciques sans générer d'anticorps qui présentent une réaction croisée avec des antigènes de tissu cardiaque humain, dans lequel cette bactérie a une variation mutationnelle provoquant une déficience nutritionnelle.

**6.** Procédé pour la préparation d'une bactérie vivante transformée non virulente, par transformation d'une bactérie non virulente avec une séquence de nucléotides hétérologue exprimant au moins un antigène immunisant de protéine M streptoccique qui est efficace pour générer des anticorps opsoniques contre des infections streptocciques sans générer d'anticorps qui présentent une réaction croisée avec des antigènes de tissu cardiaque humain, dans lequel cette bactérie hôte non virulente est choisie dans le groupe consistant en *Salmonella* et *Streptococcus*.

**7.** Procédé suivant la revendication 6, dans lequel l'hôte transformé non virulent est une *Salmonella* choisie dans le groupe consistant en *Salmonella paratyphi*, *Salmonella schottmulleri*, *Salmonella typhimurium*, *Salmonella choleraesuis*, *Salmonella montevideo*, *Salmonella newport*, *Salmonella typhim*, *Salmonella enteritidis*, *Salmonella gallinarum* et *Salmonella anatum*.

**8.** Procédé suivant la revendication 7, dans lequel la *Salmonella* est une souche non virulente transformée de *Salmonella typhimurium*.

**9.** Procédé suivant la revendication 6, dans lequel l'hôte transformé non virulent est un *Streptococcus* choisi dans le groupe consistant en *Streptococcus cremoris*, *Streptococcus faecalis*, *Streptococcus salivarius*, *Streptococcus mitior*, *Streptococcus mitis*, *Streptococcus mutans* et *Streptococcus sanguis*.

**10.** Procédé suivant la revendication 9, dans lequel l'hôte transformé non virulent est une souche de *Streptococcus Sanguis* ou de *Streptococcus mutans*.

**11.** Procédé suivant l'une quelconque des revendications 5 à 10, dans lequel la séquence de nucléotides hétérologue exprime au moins un antigène choisi dans le groupe consistant en antigènes de protéine M efficaces pour générer des anticorps opsoniques contre des streptocoques de types 5, 6 et 24.

**12.** Procédé suivant la revendication 11, dans lequel la séquence de nucléotides hétérologue exprime au moins un antigène de protéine M streptococcique qui confère une immunité contre les streptocoques de type 5.

**13.** Procédé pour préparer un vaccin, qui comprend l'incorporation en tant qu'agent vaccinant actif, d'une bactérie suivant l'une quelconque des revendications 5 à 12.

**14.** Procédé suivant l'une quelconque des revendications 1 à 4 ou 13, dans lequel ce vaccin confère une immunité systémique.

**15.** Procédé suivant l'une quelconque des revendications 1 à 4 ou 13, dans lequel ce vaccin confère une immunité locale.

**16.** Procédé suivant l'une quelconque des revendications 1 à 4 ou 13 à 15, dans lequel ce vaccin confère une immunité par administration orale.

**17.** Procédé pour la préparation d'une composition thérapeutique qui comprend le mélange d'un support acceptable du point de vue biologique et d'une bactérie transformée vivante non virulente suivant l'une quelconque des revendications 1 à 16.

**18.** Procédé suivant la revendication 17, dans lequel cette composition peut être administrée par voie orale.

FIGURE 1